# EUROPEAN PATENT SPECIFICATION

(11) **EP 3 723 506 B1**
(45) Date of publication and mention of the grant of the patent: **03.04.2024**
(21) Application number: 18815212.8
(22) Date of filing: 13.12.2018
(51) Int. Cl.: A23L 29/00, A21D 8/04, C12N 9/96, C12N 9/98, C11D 3/386, A23K 20/189, A23L 7/104

(54) **GRANULATE COMPRISING AN ENZYME, A CARRIER AND A VEGETABLE OIL**
GRANULAT MIT EINEM ENZYM, EINEM TRÄGER UND EINEM PFLANZLICHEN ÖL
GRANULÉ COMPRENANT UNE ENZYME, UN EXCIPIENT ET UNE HUILE VÉGÉTALE

(30) Priority: 14.12.2017 EP 17207296
(43) Date of publication of application: 21.10.2020
(73) Proprietor: DSM IP Assets B.V., 6411 TE Heerlen (NL)
(72) Inventor: BEEKMAN, Willem Johan, 6100 AA Echt (NL); KERKHOF, Pieter, Theodorus, 6100 AA Echt (NL); MEESTERS, Gabriel, Marinus, 6100 AA Echt (NL)
(74) Representative: dsm-firmenich IP
(86) International application number: PCT/EP2018/084680
(87) International publication number: WO 2019/115669

(56) References cited:
- EP-A2- 0 990 026
- WO-A1-2016/114648
- US-A1- 2009 317 515
- US-A1- 2010 310 720

## Description

### Field

The present invention relates to an enzyme granulate comprising a carrier and an enzyme, a process for preparing the enzyme granulate and the use of the enzyme granulate in the preparation of a food or feed product.

### Background

Enzymes granulates are widely used in the food, feed and detergent industry. Several processes for preparing enzyme-containing granulates are known. Usually a process for preparing an enzyme granulate comprises mixing an aqueous solution and a solid carrier such that the enzyme is bound to the carrier and then drying the carrier.

EP0986313 discloses a process for the preparation of an enzyme containing granulate suitable for use in an animal feed, comprising mixing an enzyme and a solid carrier comprising at least 30 % (w/w) of starch and water, mechanically processing, such as by extruding the mixture to obtain enzyme-containing granulates and drying the granulates. After drying the granulates have a water content of between 3 to 10%.

US 20020028267 discloses a method for the production of an activity stable and low dust enzyme granulate for use in the food industry, comprising synthesizing 0.01 to 20 parts by weight of enzyme or enzyme mixture, 80 to 99.99 part by weight of an organic flour type with a degree of grinding of 30% to 100%, wherein the flour type was obtained by the grinding of a flour source, using a calculated quantity of water sufficient to establish a moisture content in the moist granulate of 20 to 50 wt% and drying the granulate.

US2009/0317515 discloses solid enzyme formulations for animal feed, comprising mixtures of a salt and a support, such a wheat bran, which may further contain 0.5 or 1.0 wt% soybean oil. The solid enzyme formulation in US2009/0317515 exhibited an improvement regarding separation stability, dusting tendency and rheological behaviour

US2010/0310720 discloses a bakery enzyme composition comprising a carrier of starches or flours and optionally an oil in an amount of 0.01 to about 2.00wt%, preferably in an amount of 0.75 wt% of oil. The addition of oil aids in the prevention of segregation of components.

For baking applications, enzymes are mostly granulated with flour and the particle size of the flour is adapted to the particle size of the flour for the baking application to ensure a homogenous distribution with the flour. However, during handling of the flour serious dust problems can occur and inhaled by persons carrying out the operations, which can cause serious health problems. Addition of oil to very fine-particle enzymes may be used to dedust these particles, but it appeared that the solubility of the enzymes is reduced and thereby the effective action of the enzymes impaired. Furthermore, addition of oil to fine-particle enzyme granules can cause clotting of the granulate which may result in poor flowability and / or sieving of the granulate.

There is a need for an alternative enzyme granulate.

### Figures

**Figure 1****.** (A) Home-made apparatus for determining segregation. (B) Sampling device.
**Figure 2****.** Picture of measurement of angle of repose

### Summary

The present invention relates to an enzyme granulate comprising a carrier and an enzyme, wherein the granulate comprises from 0.015 to 0.1 wt% of vegetable oil.

The invention further relates to a process for preparing an enzyme granulate, comprising mixing a carrier with an enzyme and a composition comprising a vegetable oil, wherein the enzyme granulate comprises from 0.015 wt% to 0.1 wt% of a vegetable oil.

The invention also relates to the use of an enzyme granulate as disclosed herein to prepare a dough, a premix or a food or feed product.

### Detailed description

The present invention relates to an enzyme granulate comprising a carrier and an enzyme, wherein the granulate comprises from 0.015 to 0.1 wt% of vegetable oil. Suprisingly, it was found that an enzyme granulate that comprises from 0.015 to 0.1 wt% of vegetable oil, showed less or no segregation as compared to an enzyme granulate comprising the same carrier, but which does not comprise from 0.015 to 0.1 wt% of vegetable oil, and wherein the enzyme carrier was still flowable. Flowability can for instance be measured using Klein cups or using the angle or repose method as disclosed in the examples.

Segregation as used herein means that the one or more enzyme(s) in the enzyme granulate is (are) not homogeneously or not uniformly distributed. For instance, during storage, distribution and / or handling of (batches of) enzyme granulates segregation of enzyme granulates can occur and can be observed with the human eye. Segregation of enzyme granulates can be determined by comparing the enzymatic activity at different places in enzyme granulates after segregation.

An enzyme granulate as disclosed herein comprises a carrier and an enzyme wherein the carrier and the enzyme are a solid particle. Preferably, the carrier particle has a particle size that is similar to the particle size of the enzyme particle. The carrier particle preferably has a density that is similar to the density of the enzyme particle.

An enzyme granulate disclosed herein and prepared by a process as disclosed herein may comprise any suitable carrier, preferably an edible carrier, for instance a gluten-free carrier. A carrier as used herein is not a wax, fat or a gel. A suitable carrier as used herein is a dry carrier, for instance a suitable carrier may comprise any suitable flour. A flour may be flour from wheat, rye, barley, and / or flour from gluten-free sources such as flour from rice, potato, or corn. A carrier may also comprise maltodextrin, agglomerated maltodextrin, starch or agglomerated starch. Maltodextrin can be made from glucose syrup by methods known in the art. Maltodextrin as used herein may have a DE (dextrose equivalent) of between 3 and 20. A carrier may comprise an amount of vegetable oil, for instance from 0.5 to 25 wt% of vegetable oil, for instance from 1 to 10 wt% of vegetable oil.

The enzyme granulate as disclosed herein may comprise any suitable enzyme. An enzyme granulate as disclosed herein may comprise one or more enzymes. An enzyme as disclosed herein may for instance be an amylase, a pectinase, a protease, a lipase, a xylanase, a cellulase, an asparaginase and / or a glucose oxidase. A suitable amylase can be an alpha-amylase (E.C. 3.2.1.1.), a beta-amylase(E.C. 3.2.1.2), or a maltogenic amylase (E.C. 3.2.1.133). Pectinases include but are not limited to galacturonan 1,4-alpha-galacturonases (E.C. 3.2.1.67) and polygalacturonase (E.C. 3.2.1.15). Proteases (3.4.21.[..]) comprise endo- and exoproteases and may exhibit a preference to cleave at specific amino acid, for instance proline, glutamine etc. Lipases (E.C. 3.1.1.[..]) comprise lipolytic enzymes with different specificity, such as a phospholipase, for instance an phospholipase A1 or A2, or a phospholipase C and / or galactolipase.

(Hemi)cellulases (E.C. 3.2.1.[..]) comprise enzymes such as xylanases, arabinofuranosidases and cellulases, for instance endo- and exoglucanases.

A suitable glucose oxidase belongs to enzyme classification E.C. 1.1.3.4.

An enzyme granulate as disclosed herein comprises any suitable vegetable oil, preferably an edible vegetable oil. A suitable vegetable oil may comprise sunflower oil, palm oil, soy oil and / or canola oil.

The enzyme granulate comprises from 0.015 to 0.1 wt% of vegetable oil, for instance from 0.05 to 0.1 wt% of vegetable oil, for instance from 0.04 to 0.06 wt%, or from 0.045 to 0.08 wt%, or from 0.035 to 0.07 wt% of vegetable oil. Surprisingly, it was found that the enzyme granulate comprising from 0.015 to 0.1 wt% of vegetable oil showed less segregation than an enzyme granulate prepared with the same carrier, but not comprising from 0.015 to 0.1 wt% of vegetable oil, and still had sufficiently good flowability and did not clot.

An enzyme granulate as disclosed herein is an enzyme granulate wherein from 60% to 97%, for instance from 70% and 98%, from 80% to 99%, or from 90% to 100% of the enzyme granulates have a particle size of from 63 to 224 µm, for instance from 91% to 99.9%, from 92% to 99.8%, from 93% to 99.6%, from 94% to 99.5%, such as from 95% to 99.4% or from 95.5% to 99% of the enzyme granulates have a particle size of from 63 to 224 µm. Particle size is determined using to sieve analysis, preferaly sieve analysis according to the standardized method NPR 2244.

An enzyme granulate as disclosed herein has a bulk density of 300 to 500 kg/m³, for instance from 350 to 450 kg/m³.

An enzyme granulate as disclosed herein comprises water, for instance from 1 to 15 wt% of water, for instance from 2 to 12 wt%, such as from 3 to 10 wt%, for instance from 4 to 9 wt% of water, for instance from 5 to 8 wt% of water.

In another aspect the present invention relates to a process for preparing an enzyme granulate, comprising mixing a carrier and an enzyme and adding a composition comprising a vegetable oil, wherein the enzyme granulate comprises from 0.015 wt% to 0.1 wt% of a vegetable oil.

A process for preparing an enzyme granulate may be performed by any suitable method known in the art. An enzyme is usually first produced by fermenting a suitable microbial cell capable of producing the enzyme under conditions that allow expression of the enzyme, known to a person skilled in the art. The enzyme can be separated from the microbial cells by filtration or centrifugation, including ultrafiltration.

A process for preparing an enzyme granulate may comprise drying a liquid composition comprising the enzyme, for instance in a multi stage drying process. Multi stage drying comprises drying a material, such as a liquid composition comprising an enzyme, in physically separated sections. Drying a liquid compositition comprising an enzyme may comprise spray-drying. Spray drying is a method of producing a dry powder or dry particle from a liquid or a slurry, which is known to a person skilled in the art. Spraying drying comprises bringing a liquid composition comprising an enzyme to a temperature of between 20 and 90°C for instance between 30 and 80°C for instance between 40 and 70 °C. Spray drying may comprising bringing the liquid composition into contact with an air flow of 110°C to 190°C, for instance from 120°C to 160°C, for instance from 125°C to 145°C. During spray drying a liquid composition comprising an enzyme, a carrier, for instance wheat flour, maltodextrin, and / or rice flour may be added.

A process for preparing an enzyme granulate may further comprise fluidizing a composition comprising an enzyme in a fluidized bed subsequent to (spray) drying the enzyme. Processes of using of a fluidized bed is known to a person skilled in the art.

A process for preparing an enzyme granulate as disclosed herein comprises mixing a carrier and an enzyme. An enzyme that is mixed with a carrier in a process for preparing an enzyme granulate as disclosed herein may be in the form of a solid particle. A solid particle comprising the enzyme as disclosed herein may further comprise maltodextrin. General partical technology is for instance known from Rumpf, H. (1990) Particle technology translated by Bull, F.A., ed. Scarlett B., Chapman and Hall.

An enzyme that is mixed with a carrier in a process as disclosed herein may comprise one or more enzymes for instance an amylase, a pectinase, a protease, a lipase, a xylanase, a cellulase and / or a glucose oxidase, further disclosed herein above.

Mixing a carrier and an enzyme in a process for preparing an enzyme granulate as disclosed herein may be performed in any suitable way, using mixers known to a person skilled in the art.

A carrier that is mixed with an enzyme in a process for preparing an enzyme granulate, may be any suitable carrier as disclosed herein above. A carrier may comprise flour from wheat, rye or barley and / or a gluten-free carrier such as maltodextrin, or agglomerated maltodextrin, potato starch, or agglomerated starch, or flour from rice or corn. Preferably, the carrier comprises maltodextrin or rice flour. The carrier may comprise wheat flour.

A process for preparing an enzyme granulate as disclosed herein further comprises adding a composition comprising a vegetable oil. A composition comprising a vegetable oil may be added before, during or after mixing a carrier and an enzyme as disclosed herein. Adding a composition comprising a vegetable oil is performed in such an amount that results in an enzyme granulate comprising 0.015 to 0.1 wt% of vegetable oil, for instance from 0.05 to 0.1 wt% of vegetable oil, for instance from 0.04 to 0.06 wt% or from 0.045 to 0.08 wt%, or from 0.035 to 0.07 wt%, of vegetable oil.

A composition comprising a vegetable oil that is added to the carrier and the enzyme preferably is an edible vegetable oil and may comprise a liquid vegetable oil. A liquid vegetable oil may be vegetable oil which comprises at least 99 wt% of the vegetable oil. Alternatively, a composition comprising a vegetable oil may comprise a second carrier comprising the vegetable oil. A second carrier comprising a vegetable oil may comprise 1 to 60 wt% of vegetable oil, for instance 2 to 50 wt% of vegetable oil, for instance 5 to 40 wt%, for instance 10 to 30 wt% of vegetable oil. A second carrier may comprise maltodextrin, potato starch, wheat flour, or rice flour or flour from other sources.

A vegetable oil that is added in a process for preparing an enzyme granulate as disclosed herein may comprise sunflower oil, palm oil, soy oil and / or canola oil.

An enzyme granulate that is prepared in a process for preparing an enzyme granulate as disclosed herein may further comprise any of the embodiments disclosed herein above.

In one further aspect the present invention relates to the use of an enzyme granulate as disclosed herein in the preparation of a food or feed product, a dough or a pre-mix. Any suitable food or feed product can be prepared. A food product may be a baked product. Usually, a process for preparing a baked product comprises a step of preparing a dough. An enzyme granulate as disclosed alllows easy application and uniform distribution of the enzyme activity in the food or feed product, dough or premix.

A baked product refers to a baked food product prepared from a dough. Examples of baked products are different types of bread (white, whole-meal or rye bread), cakes, cookies and snack foods. The food product may be a gluten-free food product, for instance a gluten-free baked product. A gluten-free baked product as used herein is a product that contains at most 20 ppm gluten. Several grains and starch sources are considered acceptable for a gluten-free diet. Frequently used sources are potatoes, rice and tapioca (derived from cassava).

The present invention also relates to a dough or a premix comprising an enzyme granulate as disclosed herein.

The term "dough" is defined herein as a mixture of flour, for instance wheat flour, or flour form gluten-free sources, and other ingredients. Other ingredients as used herein comprise a lecithin source including egg, water, salt, sugar, flavours, a fat source including butter, margarine, oil and shortening, or a protein source including milk. For leavened products, a dough usually comprises baker's yeast and / or chemical leavening systems such as a combination of an acid (generating compound) and bicarbonate

A dough is firm enough to knead or roll. The dough may be fresh, frozen, prepared or parbaked. The term dough includes a batter. A batter is a semi-liquid mixture, being thin enough to drop or pour from a spoon, of one or more flours combined with liquids such as water, milk or eggs used to prepare various foods, including cake.

A premix, for instance a premix comprising an enzyme granulate as disclosed herein, usually comprises one or more further components selected from the group consisting of milk powder, gluten, granulated fat, an additional enzyme, an amino acid, a salt, oxidants (including ascorbic acid, bromate and Azodicarbonamide (ADA)), reducing agents (including L-cysteine), emulsifiers (including mono/di glycerides, monoglycerides such as glycerol monostearate (GMS), sodium stearoyl lactylate (SSL), calcium stearoyl lactylate (CSL), polyglycerol esters of fatty acids (PGE) and diacetyl tartaric acid esters of mono- and diglycerides (DATEM), gums (including guargum and xanthangum), flavours, acids (including citric acid, propionic acid), starch, modified starch, gluten, humectants (including glycerol) and preservatives.

The present invention also relates to a process for preparing a food or feed product comprising mixing an enzyme granulate as disclosed herein with suitable further food or feed ingredients and preparing the food or feed product. Any suitable ingredients may be used to prepare a food or feed product known to a person skilled in the art. In the event the food product is a baked product as disclosed herein above, the enzyme granulate may be mixed with flour, and other ingredients, for instance as disclosed herein above, preparing a dough and baking the dough to prepare a baked product. The present invention also relates to a process for preparing a dough, or a premix comprising mixing the enzyme granulate as disclosed herein with any suitable ingredient for preparing a dough or premix, such a flour, yeast and / or water.

### EXAMPLES

### MATERIALS & METHODS

Wheat flour (Flour TM80) and Rice flour (Heat Treated Rice flour) were obtained from (Westhove, France).

Maltodextrin (DE≤20) (Diluant White) was prepared by diluting a 60 wt% maltodextrin syrup (Roclys^{®} C 1967 S, Roquette, France) water to 40 wt% maltodextrin syrup and dissolving 1.7 wt% NaCl. The liquid is heat shocked at 90°C to decrease microbial growth. The liquid is kept at elevated temperatures (>40°C) until drying.

Sunflower oil was from (Levo, batch no. 2363).

DW/oil is a mixture of Diluant White (maltodextrin) with 25 wt% sunflower oil.

Diluant White SF is a mixture of Diluant White with 1.5 wt% of sunflower oil.

Dedust, IFT (AB Mauri) is a mixture of potato starch, 43 wt%, wheat flour, 22 wt% and sunflower oil, 35 wt%.

### Enzymes

Panamore^{®} Golden GF, BakeZyme^{®} FXP GF 1500, Pectinase MG, and BakeZyme^{®} Go Classic GF 10.000 were obtained from DSM.

Bakezyme ^{®} FXP GF 1500 is a fungal xylanase from *Rasamsonia emersonii,* standardized at 1500 NXTU/g.

Panamore ^{®} Golden GF is a lipase produced in an *Aspergillus* sp. standardized at 2750 DLU/g. BakeZyme^{®} Go Classic 10.000 is a glucose oxidase obtained from *Aspergillus niger* standardized at 10000 SRU/g on wheat flour

Pectinase MG contains a polygalacturonase derived from *Aspergillus* niger standardized at 250000 AVJP/g.

### Enzyme activity assays

### Glucose oxidase

Glucose oxidase activity is determined in an assay in which gluconic acid formed is titrated. 1 ml of diluted glucose oxidase is added to 25 ml of preheated 30 g/l glucose monohydrate solution at 35 degrees C. Sample dilutions and substrate are prepared in 50 mM HAc buffer at pH 5.1, containing 50 mM NaCl. After 15 minutes incubation at 35 degrees C, the addition of 10 ml 0.1 N NaOH the terminated the reaction, at the same time neutralizing the gluconic acid formed. Excess NaOH is titrated with 0.05 M HCl. The difference in HCl consumption between a sample and blank run is a measure for the amount of glucose oxidase activity. One glucose oxidase unit (SRU) is defined as the amount of enzyme needed to oxidize 3 mg of glucose to gluconic acid under conditions of the assay.

### Lipase

Lipase activity may be expressed in pNP (4-nitrophenol) Units. One Unit (DLU) is defined as the amount of enzyme that liberates one micromole of 4-nitrophenol per minute under the conditions of the test.

The principle of the assay is as follows: A mix of enzyme, buffer, and substrate solution is incubated at 25 °C for 30 minutes. During the incubation time, absorbance at 348 nm is measured.

Substrate solution was prepared as follows: An 8.0 mM solution of the chromogenic substrate in 2-propanol was made. Subsequently, 3.5 mL of this solution was added to 46.5 mL 100 mmol/L sodium acetate buffer pH 5.5 containing 1% Triton X-100, under vigorous stirring. Substrate was pNP-palmitate (N2752, Sigma Aldrich).

Enzyme was diluted in 100 mmol/L sodium acetate buffer pH 5.5 containing 1% Triton X-100, such that the absorbance increase after 30 minutes is less than 1.0. Reaction was started by mixing 10 microliter of diluted enzyme solution with 240 microliter of substrate solution in a microtiter plate. 200 microliter of this mixture was added to a fresh microtiter plate, placed in a TECAN Infinite M1000 microtiter plate reader, temperature is kept at 25 °C, and the change in absorption of the mixture was measured for 30 minutes at 348 nm (isosbestic point of 4-nitrophenol). The blank sample was prepared by adding 10 microliter of sodium acetate buffer pH 5.5 instead of enzyme solution to the substrate solution and then following the steps identical as described above for the enzyme reaction. A calibration line was produced from 4-nitrophenol dissolved in 100 mmol/L sodium acetate buffer pH 5.5 containing 1% Triton X-100.

The absorbance of the samples was plotted against the time. The slope was determined over the linear part of the absorbance measurement. The slope of the enzyme reaction was corrected by subtracting the slope of the blank reaction. Subsequently activity was calculated relative to the slope of the calibration line.

### Pectinase

The reduction of viscosity of a solution of about 15.5 g/L methylated pectin (degree of methylation>85%) caused by pectinase enzyme activity is measured at pH 3.85 and 50°C using an Ubbelohde viscometer (Type C). The reduction of viscosity is a measure for the enzyme activity. Pectinase activity is expressed in AVJP, wherein one unit AVJP is the enzyme quantity in 1 ml enzyme solution which induces a variation of viscosity with a speed from which the apparent constant is 0.00027 / minute under the conditions of the test.

### Xylanase

Activity of endoxylanase is measured by the release of para-nitrophenol (pNP) from P-Nitrophenyl-beta-D-xylopyranoside (pNP-X, Sigma-Aldrich N2132), using a Konelab Arena 30 clinical analyzer. Enzyme samples are incubated with the substrate for 16 minutes and 40 seconds, after which the reaction is terminated (and color developed) by the addition of an alkaline stop solution.

The pNP-X substrate is prepared by dissolving 100 mg pNP-X in 50 ml sodium acetate buffer (30 mM) pH 4.5. Enzymatic samples are diluted to a range between 0.25 - 1.5 NTXU/ml in 30 mM sodium acetate buffer to which 0.2% Triton X-100 is added.

156 µl substrate is preheated in the analyser for 5 minutes at 37°C, after which the reaction is started by the addition of 13 µl of suitably diluted sample. After the incubation, 80 µl of a 300 mM sodium carbonate solution is added, and the resulting absorbance increase because of enzymatic activity is measured at 405 nm. Activity is calculated from a pNP calibration (using a pNP solution of known concentration) performed on the same equipment, with the same reagents.

One NTXU is defined as the amount of enzyme that liberates 0.06 µmol p-nitrophenol per minute under the conditions of the assay (pH 4.5, 37°C).

### Segregation

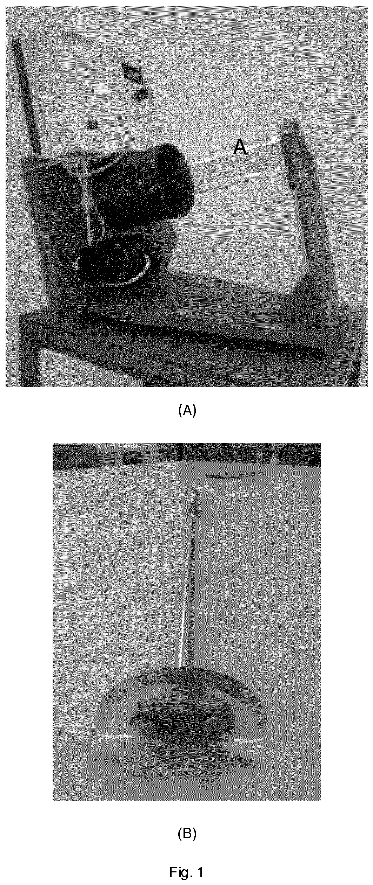

Segregation was measured in a home-made apparatus consisting of a glass tube (dimensions: length = 35 cm, diameter = 6.5 cm) which is put in a holder under an angle of 30° (see Figure 1). An amount of 150 g enzyme granulate was put into the tube and subsequently the tube was rotated with such a speed that causes that the material flows gently. After at least 30 minutes rotation, samples were taken from the top fraction and from the bottom fraction using the dedicated sampling device (see Figure 1) and enzyme activity was determined as described above.

For the segregation test in Example 3, the glass cylinder was rotated under a 15° angle and an amount of 200 g enzyme granulate was put into the tube.

### Determination of particle size

For the determination of particle size of the enzyme granulates the standard NPR2244 Test Sieves Application was applied, using a sieving machine (Retsch sieve, type RS200 digit), with sieves having a diameter of 200 mm. The sieves had a pore diameter of 1000 µm, 224 µm and 63 µm. 25 g of enzyme granulate was weighed into the stacked sieves. During 10 min. the sieve deck was vibrated. The separate sieves were weighed and the percentage of the residue of the starting material on each sieve was calculated.

For the experiments in Example 3, the particle sizes of the enzyme granulates were determined in sieves (diameter of 200 mm) having a pore diameter of 250 µm, 224 µm, 180 µm, 150 µm, 125 µm, 90 µm and 63 µm, using an amount of 50 g of sample load. Sieving was performed as described above.

### Determination of flowability

### Klein cups

The flowability was determined using so-called Klein cups, a glass tube ending conical with a defined opening in the bottom, having a diameter of 2.4 mm, 5 mm, 8 mm, 12 mm or 18 mm.

The cup is filled for about 75% with enzyme granulate. When the enzyme granulate flows through the tube without help like ticking on the wall, that is the diameter for that flowability. The smaller the diameter, the better the flowability: a diameter of 2.4 mm is excellent, 5 mm is good, 8 mm is amply sufficient, 12 mm is just sufficient, and 18 mm is poor.

### Angle of repose

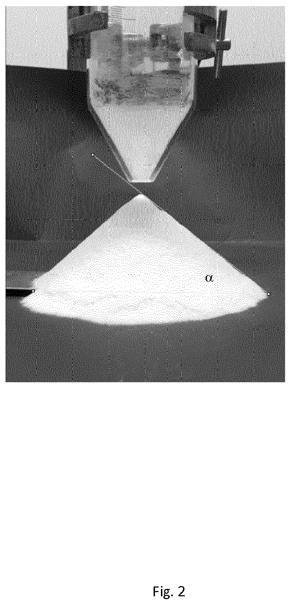

Measurement of the angle of repose of a powder is based on the principle that a powder flows from a small aperture at the bottom of a glass container to form a conical shaped mountain (P. Schuck et al., Analytical Methods for Food and Dairy Powders (2012), Wiley-Blackwell, ISBN 978-0-470-65598-6. The angle of the slope of the "mountain" with the base line is a measure for the flowability. A photo is taken of the produced conus (See a sample picture Figure 2). This photo is analyzed using the software package: imaged 1.51j8.

The values for categorizing the flowability are given in Table 1 (from Table 8.1, page 138, from P. Schuck et al., Analytical Methods for Food and Dairy Powders (2012), Wiley-Blackwell, ISBN 978-0-470-65598-6

**Table 1 Values for categorizing the flowability**

| **Degree** | **Flowability performance** |
|---|---|
| <25 - 30 | Excellent |
| 31 - 35 | Good |
| 36 - 40 | Fair |
| 41 - 45 | Passable |
| 46 - 55 | Poor |
| 56 - 65 | Very poor |
| 66 - 90 | Very very poor |

### EXAMPLE 1

Enzyme granulates were made by mixing a carrier with an enzyme in a Turbula mixer (Turbula T2F) for 30 min. The weight percentage of carrier and enzyme granulate of lipase, xylanase and pectinase were as indicated in Table 2.

**Table 2. Weight percentage of crude granulate and carrier**

| **Enzyme** | **Product name** | **Crude granulate in blend (%)** | | **Carrier in blend (%)** |
|---|---|---|---|---|
| Lipase | Panamore^{®} Golden GF | | 9.2 | 90.8 |
| Xylanase | Bakezyme^{®} FXP GF | | 18.5 | 81.5 |
| Pectinase | Pectinase MG | | 17.9 | 82.1 |

Bakezyme^{®} FXP GF xylanase and Pectinase MG pectinase were granulated with rice flour and Diluant White and both Dedust GF and DW/oil were used as a repressor for segregation (0.05% added oil).

Panamore^{®} Golden GF lipase was granulated with rice flour and Dedust GF was used as repressor for segregation (0.05 wt% added oil).

Bakezyme^{®} GO Classic glucose oxidase was granulated on wheat flour (Flour TM80). DW/oil was mixed with Bakezyme^{®} GO Classic in such an amount that a wt% of oil in the final enzyme granulate of 0.05%, 0.1%. 0.5%, 1% and 2% was obtained.

After mixing, the enzyme granulates were treated in the segregation tester as described above. The start mixture of enzyme granulates, and the bottom and top fractions of the enzyme granulate after segregation were sampled and analysed for the relevant enzyme activity.

The results in Tables 3 to 5 show that the addition of 0.05 wt% of oil reduced the difference of enzyme activity between the top and bottom fractions of the enzyme granulate comprising maltodextrin (Diluant white) or rice flour as a carrier.

Table 6 shows that addition of 0.05 wt% and 0.5 wt% of oil reduced the difference of enzyme activity of an enzyme granulate comprising wheat flour as a carrier as compared to an enzyme granulate prepared from the same carrier with no oil.

### Xylanase

**Table 3. Xylanase activity (%) of enzyme granulate in samples from the top and the bottom of a segregation tube after segregation test, relative to the xylanase activity of the enzyme granulate before segregation.**

| **Carrier** | **Xylanase activity (%)** | |
|---|---|---|
| | **top** | **bottom** |
| Rice flour | 101% | 89% |
| Rice flour + Dedust (0.05% oil) | 100% | 92% |
| Rice flour + DW/oil (0.05% oil) | 106% | 92% |
| Diluant White | 220% | 66% |
| Diluant White + Dedust (0.05% oil) | 114% | 97% |
| Diluant White + DW/oil (0.05% oil) | 104% | 91% |

### Lipase

**Table 4. Lipase activity (%) of enzyme granulate in samples from the top and the bottom of a segregation tube after segregation test, relative to the lipase activity of the enzyme granulate before segregation.**

| | **Lipase activity (%)** | |
|---|---|---|
| **Carrier** | **top** | **bottom** |
| Rice flour | 119 | 94 |
| Rice flour + Dedust (0.05% oil) | 109 | 88 |
| Diluant White | 260 | 42 |
| Diluant White + Dedust (0.05% oil) | 102 | 92 |

### Pectinase

**Table 5. Pectinase activity (%) of enzyme granulate in samples from the top and the bottom of a segregation tube after segregation test, relative to the pectinase activity of the enzyme granulate before segregation.**

| | **Pectinase activity (%)** | |
|---|---|---|
| **Carrier** | **Top** | **bottom** |
| Diluant White | 145 | 102 |
| Diluant White + Dedust (0.05% oil) | 105 | 100 |
| Diluant White + DW/oil (0.05% oil) | 102 | 103 |

### Glucose oxidase

**Table 6. Glucose oxidase activity (%) of enzyme granulate in samples from the top and the bottom of a segregation tube after segregation test, relative to the glucose oxidase activity of the enzyme granulate before segregation.**

| | **Glucose oxidase activity (%)** | | |
|---|---|---|---|
| Glucose oxidase on wheat flour | **Top** | **Bottom** | |
| no oil addition | 174.3 | | 70.6 |
| , 0.05% oil | 139.7 | | 86.5 |
| 0.1% oil | 113.2 | | 96.9 |
| , 0.5% oil | 101.0 | | 103.6 |

### EXAMPLE 2

### 2.1. Particle size distribution

Particle size of the granulates comprising rice flour or maltodextrin (Diluant White) was determined using the sieving test as described above. The particle size was determined without addition of enzyme. The results in Table 7 show that the particle size distribution (by sieve analysis) is not affected by the addition of 0.05% of oil in the granulate.

**Table 7. Particle size distribution of granulates prepared from different carriers**

| **Carrier** | **% >224 µm** | **% 224 - 63 µm** | **% < 63 µm** |
|---|---|---|---|
| Rice flour | 0.4 | 98.8 | 1.2 |
| Rice flour + Dedust (0.05 % oil) | 0.4 | 98.4 | 1.6 |
| Rice flour + DW/oil (0.05 % oil) | 0.4 | 98.4 | 1.2 |
| Diluant White | 3.2 | 94.4 | 2.8 |
| Diluant White + Dedust (0.05 % oil) | 3.6 | 96.0 | 1.2 |
| Diluant White + DW/oil (0.05 % oil) | 2.4 | 96.4 | 0.8 |

T

### 2.2. Flowability

The flowability of the different granulates was determined using Klein Cups as disclosed above. The flowability of the granulates comprising rice flour or maltodextrin (Diluant White) without enzyme addition was tested. The results in Table 8 show that the granulates with 0.05% oil still have a flowability that is good or amply sufficient.

**Table 8. Flowability of granulates prepared from different carriers**

| | **Klein cup** | |
|---|---|---|
| **Carrier** | **Diameter (mm)** | **meaning** |
| Rice flour | 5 | good |
| Rice flour + Dedust (0.05 % oil) | 8 | amply sufficient |
| Rice flour + DW/oil (0.05 % oil) | 8 | amply sufficient |
| Diluant White | 2.4 | very good |
| Diluant White + Dedust (0.05 % oil) | 5 | good |
| Diluant White + DW/oil (0.05 % oil) | 5 | good |

The flowability of Bakezyme^{®} GO Classic glucose oxidase enzyme granulates on wheat flour comprising 0, 0.05, 0.1, 0.5, 1 and 2 wt% of oil was also tested. The results in Table 9 show that the flowability of glucose oxidase enzyme granulates on wheat flour to which up to 0.5 wt% oil was added was still sufficient.

**Table 9. Flowability of Bakezyme^{®} GO Classic glucose oxidase granulates with different amounts of added oil.**

| **Glucose oxidase granulate** | | **Klein cup** | |
|---|---|---|---|
| **% oil** | **Diameter (mm)** | | **meaning** |
| 0 | | 5 | good |
| 0.05 | | 5 | good |
| 0.1 | | 5 | good |
| 0.5 | | 12 | just sufficient |
| 1 | not through 18 | | no flow |
| 2 | not through 18 | | no flow |

### EXAMPLE 3

Enzyme granulates from Bakezyme^{®} BXP xylanase and Panamore^{®} Golden lipase were made by mixing crude granulate of the enzymes with Diluant White, and appropriate amounts of Diluant White SF in a Turbula mixer (Turbula T2F) for 30 min. The amount of oil in wt% in the enzyme granulates containing xylanase or lipase was 0, 0.02, 0.05, 0.1 and 0.75 wt%.

### 3.1. Sieve analysis

For the sieve analysis Bakezyme^{®} BXP xylanase and Panamore^{®} Golden lipase granulates with 0, 0.05 and 0.75 wt% oil were sieved according to the sieve analysis method according to NPR 2244 as described above.

The results in Table 10 and Table 11 show that the xylanase and lipase granulates having 0.75 wt% oil had more than 50% of particles having a size of more 250 µm. The particle size distribution of granulates having 0.05 wt% of oil was similar to the particle size distribution of granulates wherein no oil was added.

**Table 10. Particle size distribution by sieve analysis of Bakezyme^{®} BXP xylanase granulate with 0, 0.05 wt% and 0.75 wt% oil**

| **Sieve pore diameter (µm)** | **no oil (%)** | **0.05 wt% oil (%)** | **0.75 wt% oil (%)** |
|---|---|---|---|
| <63 | 0.4 | 0.4 | 0.0 |
| 63-90 | 10.0 | 9.4 | 0.0 |
| 90-125 | 13.4 | 17.8 | 0.1 |
| 125-150 | 18.0 | 33.7 | 0.2 |
| 150-180 | 44.3 | 29.1 | 6.1 |
| 180-224 | 13.9 | 8.5 | 10.2 |
| 224-250 | 1.3 | 1.4 | 29.2 |
| >250 | 0.5 | 0.6 | 56.8 |

**Table 11. Particle size distribution by sieve analysis of Panamore^{®} Golden lipase granulates with 0, 0.05 wt% and 0.75 wt% oil**

| **Sieve pore diameter (µm)** | **no oil (%)** | **0.05 wt% oil (%)** | **0.75 wt% oil (%)** |
|---|---|---|---|
| <63 | 6.2 | 0.3 | 0.2 |
| 63-90 | 13.1 | 10.1 | 0.1 |
| 90-125 | 25.5 | 19.4 | 0.2 |
| 125-150 | 37.5 | 54.8 | 0.3 |
| 150-180 | 10.6 | 9.6 | 2.6 |
| 180-224 | 5.3 | 5.0 | 12.4 |
| 224-250 | 1.1 | 1.1 | 28.2 |
| >250 | 0.8 | 0.9 | 57.1 |

### 3.2. Flowability

The flowability of the Bakezyme^{®} BXP xylanase and Panamore^{®} Golden lipase granulates containing 0, 0.02 wt%, 0.05 wt%, 0.10 wt% and 0.75 wt% was measured using the angle of repose method as described above.

The results in Table 12 and 13 (average of 8 measurements) show that with increasing oil content the flowability of the granulates decrease, but up to 0.1 wt% of oil the granulates are still flowable. The xylanase and lipase granulates containing 0.75 wt% of oil did not flow through the aperture at the bottom of the glass cylinder in the angle of response method.

**Table 12. Angle of repose flowability of Bakezyme^{®} BXP xylanase granulates with different oil content**

| **Oil content (wt%)** | **0** | **0.02** | **0.05** | **0.10** |
|---|---|---|---|---|
| Angle of repose | 40.4 | 44.0 | 50.1 | 56.2 |
| Flowability | FAIR | PASSABLE | POOR | VERY POOR |

**Table 13. Angle of repose flowability of Panamore^{®} Golden lipase granulates with different oil content**

| **Oil content (wt%)** | **0** | **0.02** | **0.05** | **0.10** |
|---|---|---|---|---|
| Angle of repose | 33.4 | 54.3 | 58.3 | 56.8 |
| Flowability | GOOD | POOR | VERY POOR | VERY POOR |

### 3.3. Segregation of granulates

Bakezyme ^{®} BXP xylanase and Panamore^{®} Golden lipase granulates containing 0, 0.02 wt%, 0.05 wt%, 0.10 wt% and 0.75 wt% of oil, were treated in the segregation tester as described above.

After the segregation test, samples were taken at the top, middle and bottom of the segregation tube and xylanase and lipase activity was determined as described above.

The results in Table 14 and Table 15 show the percentage of xylanase and lipase activity relative to the xylanase and lipase activity respectively of the granulates before segregation. Addition of 0.02 wt% to 0.75 wt% of oil suppressed segregation.

**Table 14. Xylanase activity (%) of enzyme granulate in samples taken from the top, middle and bottom of a segregation tube after segregation test, relative to the xylanase activity of the enzyme granulate before segregation**

| **Oil addition Wt%** | **Top %** | **Middle %** | **Bottom %** |
|---|---|---|---|
| 0 | 107 | 80 | 86 |
| 0.02 | 96 | 104 | 111 |
| 0.05 | 109 | 99 | 108 |
| 0.10 | 102 | 106 | 103 |
| 0.75 | 98 | 99 | 101 |

**Table 15. Lipase activity (%) of enzyme granulate in samples taken from the top, middle and bottom of a segregation tube after segregation test, relative to the phospholipase activity of the enzyme granulate before segregation**

| **Oil addition Wt%** | **Top %** | **Middle %** | **Bottom %** |
|---|---|---|---|
| 0 | 196 | 81 | 108 |
| 0.02 | 97 | 102 | 100 |
| 0.05 | 99 | 95 | 79 |
| 0.10 | 106 | 94 | 106 |
| 0.75 | 101 | 101 | 93 |

### Conclusion

The examples show that enzyme granulates containing 0.02, 0.05, and 0.1 wt% of oil showed reduced segregation and were still flowable. An enzyme granulate comprising 0.75 wt% of oil also showed reduced segregation, but the granulate did not flow, and more than 50% of the granulate having 0.75 wt% of oil had a particle size of more than 250 µm.

## Claims

1. An enzyme granulate comprising a carrier and an enzyme, and wherein the granulate comprises from 0.015 to 0.1 wt% of vegetable oil.

2. The enzyme granulate according to claim 1, wherein the carrier comprises wheat flour, maltodextrin, agglomerated maltodextrin, or rice flour.

3. The enzyme granulate according to claim 1 or 2, wherein the enzyme is an amylase, pectinase, protease, a lipase, a xylanase, a cellulase, asparginase and / or a glucose oxidase.

4. The enzyme granulate according to any one of the claims 1 to 3, wherein the vegetable oil comprises sunflower oil, palm oil, soy oil and / or canola oil.

5. The enzyme granulate according to any one of the claims 1 to 4, wherein from 60% to 100% of the granulate has a particle size of between 63 to 224 µm based on sieve analysis.

6. A process for preparing an enzyme granulate, comprising mixing a carrier with an enzyme and adding a composition comprising a vegetable oil, wherein the enzyme granulate comprises 0.015 wt% to 0.1 wt% of the vegetable oil.

7. The process according to claim 6, wherein adding a composition comprising a vegetable oil is performed before, during or after the mixing of a carrier with an enzyme.

8. The process according to claim 6 or 7, wherein the composition comprising the vegetable oil is a liquid vegetable oil

9. The process according to claims 6 or 7, wherein the composition comprising a vegetable oil is a second carrier comprising a vegetable oil.

10. The process according to any one of the claims 6 to 9, wherein the carrier that is mixed with the enzyme comprises wheat flour, maltodextrin, agglomerated maltodextrin, or rice flour.

11. The process according to claim 9, wherein the second carrier comprises wheat flour, maltodextrin, agglomerated maltodextrin, or rice flour.

12. The process according to any one of the claims 6 to 11, wherein the vegetable oil comprises sunflower oil, palm oil, soy oil and / or canola oil.

13. A process for preparing a food or feed product, a dough or a premix, comprising mixing an enzyme granulate according to any one of the claims 1 to 5 with suitable further ingredients for preparing a food or feed product, a dough or a premix.

14. Use of an enzyme granulate according to claims 1 to 5 in the preparation of a food or feed product, a dough or a premix.

15. Use according to claim 14 or process according to claim 13, wherein the food product is a baked product.

## Patentansprüche

1. Enzymgranulat, umfassend einen Träger und ein Enzym, wobei das Granulat von 0,015 bis 0,1 Gew.-% Pflanzenöl umfasst.

2. Enzymgranulat gemäß Anspruch 1, wobei der Träger Weizenmehl, Maltodextrin, agglomeriertes Maltodextrin oder Reismehl umfasst.

3. Enzymgranulat gemäß Anspruch 1 oder 2, wobei das Enzym eine Amylase, Pectinase, Protease, eine Lipase, eine Xylanase, eine Cellulase, Asparaginase und/oder eine Glucoseoxidase ist.

4. Enzymgranulat gemäß einem der Ansprüche 1 bis 3, wobei das Pflanzenöl Sonnenblumenöl, Palmöl, Sojaöl und/oder Rapsöl umfasst.

5. Enzymgranulat gemäß einem der Ansprüche 1 bis 4, wobei von 60 % bis 100 % des Granulats eine Partikelgröße zwischen 63 und 224 µm auf der Grundlage von Siebanalyse aufweisen.

6. Verfahren zur Herstellung eines Enzymgranulats, umfassend Mischen eines Trägers mit einem Enzym und Zugeben einer Zusammensetzung, die ein Pflanzenöl umfasst, wobei das Enzymgranulat von 0,015 bis 0,1 Gew.-% an dem Pflanzenöl umfasst.

7. Verfahren gemäß Anspruch 6, wobei Zugeben einer Zusammensetzung, die ein Pflanzenöl umfasst, vor, während oder nach dem Mischen eines Trägers mit einem Enzym durchgeführt wird.

8. Verfahren gemäß Anspruch 6 oder 7, wobei die Zusammensetzung, die das Pflanzenöl umfasst, ein flüssiges Pflanzenöl ist.

9. Verfahren gemäß Anspruch 6 oder 7, wobei die Zusammensetzung, die ein Pflanzenöl umfasst, ein zweiter Träger ist, der ein Pflanzenöl umfasst.

10. Verfahren gemäß einem der Ansprüche 6 bis 9, wobei der Träger, der mit dem Enzym gemischt wird, Weizenmehl, Maltodextrin, agglomeriertes Maltodextrin oder Reismehl umfasst.

11. Verfahren gemäß Anspruch 9, wobei der zweite Träger Weizenmehl, Maltodextrin, agglomeriertes Maltodextrin oder Reismehl umfasst.

12. Verfahren gemäß einem der Ansprüche 6 bis 11, wobei das Pflanzenöl Sonnenblumenöl, Palmöl, Sojaöl und/oder Rapsöl umfasst.

13. Verfahren zur Herstellung eines Lebensmittel- oder Futterprodukts, eines Teigs oder einer Vormischung, Umfassend Mischen eines Enzymgranulats gemäß einem der Ansprüche 1 bis 5 mit geeigneten weiteren Inhaltsstoffen zur Herstellung eines Lebensmittel- oder Futterprodukts, eines Teigs oder einer Vormischung.

14. Verwendung eines Enzymgranulats gemäß Ansprüchen 1 bis 5 bei der Herstellung eines Lebensmittel- oder Futterprodukts, eines Teigs oder einer Vormischung.

15. Verwendung gemäß Anspruch 14 oder Verfahren gemäß Anspruch 13, wobei das Lebensmittelprodukt eine Backware ist.

## Revendications

1. Granulé d'enzyme comprenant un support et une enzyme, et le granulé comprenant de 0,015 à 0,1 % en poids d'huile végétale.

2. Granulé d'enzyme selon la revendication 1, le support comprenant une farine de blé, une maltodextrine, une maltodextrine agglomérée ou une farine de riz.

3. Granulé d'enzyme selon la revendication 1 ou 2, l'enzyme étant une amylase, une pectinase, une protéase, une lipase, une xylanase, une cellulase, une asparaginase et/ou une glucose oxydase.

4. Granulé d'enzyme selon l'une quelconque des revendications 1 à 3, l'huile végétale comprenant l'huile de tournesol, l'huile de palme, l'huile de soja et/ou l'huile de canola.

5. Granulé d'enzyme selon l'une quelconque des revendications 1 à 4, de 60 % à 100 % du granulé ayant une taille de particule comprise entre 63 et 224 µm sur la base d'une analyse sur tamis.

6. Procédé pour la préparation d'un granulé d'enzyme, comprenant le mélange d'un support avec une enzyme et l'ajout d'une composition comprenant une huile végétale, le granulé comprenant de 0,015 à 0,1 % en poids de l'huile végétale.

7. Procédé selon la revendication 6, l'ajout d'une composition comprenant une huile végétale étant réalisé avant, pendant ou après le mélange d'un support avec une enzyme.

8. Procédé selon la revendication 6 ou 7, la composition comprenant l'huile végétale étant une huile végétale liquide.

9. Procédé selon la revendication 6 ou 7, la composition comprenant une huile végétale étant un deuxième support comprenant une huile végétale.

10. Procédé selon l'une quelconque des revendications 6 à 9, le support qui est mélangé avec l'enzyme comprenant une farine de blé, une maltodextrine, une maltodextrine agglomérée ou une farine de riz.

11. Procédé selon la revendication 9, le deuxième support comprenant une farine de blé, une maltodextrine, une maltodextrine agglomérée ou une farine de riz.

12. Procédé selon l'une quelconque des revendications 6 à 11, l'huile végétale comprenant l'huile de tournesol, l'huile de palme, l'huile de soja et/ou l'huile de canola.

13. Procédé pour la préparation d'un produit alimentaire ou d'un produit alimentaire pour animaux, d'une pâte ou d'un prémélange, comprenant le mélange d'un granulé d'enzyme selon l'une quelconque des revendications 1 à 5 avec des ingrédients supplémentaires appropriés pour la préparation d'un produit alimentaire ou d'un produit alimentaire pour animaux, d'une pâte ou d'un prémélange.

14. Utilisation d'un granulé d'enzyme selon les revendications 1 à 5 dans la préparation d'un produit alimentaire ou d'un produit alimentaire pour animaux, d'une pâte ou d'un prémélange.

15. Utilisation selon la revendication 14 ou procédé selon la revendication 13, le produit alimentaire étant un produit cuit.
